# EUROPEAN PATENT APPLICATION

(11) **EP 3 513 679 A1**
(43) Date of publication of application: **24.07.2019**
(21) Application number: 17850543.4
(22) Date of filing: 24.07.2017
(51) Int. Cl.: A43D 1/02, G01B 11/24

(54) **DEVICE FOR ACQUIRING DATA FOR DESIGNING WOODEN PATTERN**

(30) Priority: 14.09.2016 JP 2016179448
(71) Applicant: Millimeter, Inc., Tokyo 141-0022 (JP)
(72) Inventor: KASUYA, Takashi, Tokyo 141-0022 (JP); IGURO, Tateaki, Tokyo 141-0022 (JP)
(74) Representative: Zacco Denmark A/S
(86) International application number: PCT/JP2017/026602
(87) International publication number: WO 2018/051637

(57) **Abstract**

The present invention provides an apparatus for acquiring data for designing a last capable of accurately measuring a three-dimensional shape of a sole of a foot that is curved as if a footwear is being worn thereon. The apparatus for acquiring data for designing a last includes a placement section 2 for placing feet F and F' thereon and an imaging means 3 arranged around the placement section 2. The placement section 2 includes a heel rest 12 for placing the heel of the foot F or the foot F' thereon, thereby enabling accurate measurement of the data of the sole that is curved in such a state as if footwear having a heel such as a pump is being worn thereon , without moving the foot during the measurement.

## Description

### TECHNICAL FIELD

The present invention relates to an apparatus for acquiring data for designing a last, which measures the shape of a foot to produce a shoe-making last.

### BACKGROUND ART

As an apparatus for acquiring data for designing a last of this type, there has been known, for example, an apparatus disclosed in Patent Document 1. In this apparatus, images are taken by two optical cameras, and data are taken into a digitizer using a three-dimensional shape measuring device in which optical techniques such as an optical cutting method are combined with software techniques such as three-dimensional graphics. Then, the data are input, as an video signal, into an I/F video board via a control board, processed at a phase calculation processing section and a noise correction processing section, and thus three-dimensional data are output. There has been, however, a problem that the outline of a foot has to be extracted from the images taken by the two optical cameras and three-dimensional measurement results, and parts of the foot have to be estimated based on the results.

Patent Document 2 discloses a three-dimensional measurement apparatus of foot including: a transparent plate for placing a foot to be measured thereon; a slit light projection device for projecting slit light on an upper part of an instep of the foot; an image taking device arranged above the instep of the foot with a certain distance away from the slit light projection device; an image processing device configured to take images so as to recognize only reflection of the slit light among the images taken by the image taking device and to calculate the shortest height from the transparent plate to a certain spot on the instep on which the slit light strikes; and a camera arranged under the transparent plate to take two-dimensional images of the whole hand or foot. This three-dimensional measurement apparatus of foot also includes an arithmetic device that performs image correction such that the two-dimensional image data taken by the camera and the three-dimensional shape data obtained by the image taking device and the image processing device are combined with each other while ensuring consistency; and a medium that stores data obtained by the arithmetic device. Performing image recognition from the bottom side of the foot in addition to from the instep side thereof makes it possible to utilize the features of the bottom side of the foot as a determining condition for forming a three-dimensional image of the foot.

### PRIOR ART DOCUMENTS

### Patent Documents

Patent Document 1: Japanese Patent Application Laid-Open No. 2003-085416
Patent Document 2: Japanese Patent Application Laid-Open No. 2004-003086

### SUMMARY OF THE INVENTION

### Problems to be solved by the invention

According to such apparatus for acquiring data for designing a last, however, since a foot is placed on a flat measurement table, the shape of the foot becomes so-called "flat-footed" one. Particularly, when a user's footwear is one having a heel such as a pump, it is difficult, in view of the structure of a foot itself, to accurately produce data on the foot on which a footwear is being worn, from data on the foot itself that is in such a flat-footed state, leading to difficulties in acquiring data for producing comfortable footwear that fits to the user's feet, from measurement data of the flat-footed one.

Further, even if a foot is measured with a heel being raised from the floor in such a state as if footwear were worn on the foot, it is difficult to continue to take measurements without moving the foot during the measurement. Particularly, it has been difficult to accurately acquire data of a sole that is curved as if footwear were being worn thereon.

In light of the foregoing problems, it is an object of the present invention to provide an apparatus for acquiring data for designing a last that takes foot measurements in such a state as if footwear were being worn thereon so that one is capable of accurately measuring a three-dimensional shape of a sole that is curved as if footwear were being worn thereon.

### Means for solving the problems

An apparatus for acquiring data for designing a last according to a first aspect of the present invention includes:
a main body having a flat plate shape; a placement section arranged on an upper surface of the main body, the placement section placing a foot thereon; a first imaging section arranged around the placement section, the first imaging section imaging a whole circumference of the foot; a second imaging section disposed on the main body, the second imaging section imaging a vicinity of an instep of the foot; a third imaging section disposed on the main body, the third imaging section imaging a vicinity of an ankle of the foot; and a heel rest arranged on the placement section, the heel rest placing a heel thereon.

Further, according to the apparatus for acquiring data for designing a last of a second aspect of the present invention, the first imaging section images the foot placed on the placement section at such an angle that the first imaging section is turned upward.

The apparatus for acquiring data for designing a last according to a third aspect of the present invention further includes an entire illumination for illuminating the circumference of the placement section.

The apparatus for acquiring data for designing a last according to a fourth aspect of the present invention further includes a sole illumination for illuminating a sole.

According to the apparatus for acquiring data for designing a last according to a fifth aspect of the present invention, the placement section is composed of the heel rest and a sole illumination table having the sole illumination.

According to the apparatus for acquiring data for designing a last according to a sixth aspect of the present invention, the sole illumination table is formed to be larger than an area of a sole, and is provided with an illumination table main body that transmits light from the sole illumination.

According to the apparatus for acquiring data for designing a last according to a seventh aspect of the present invention, the illumination table main body is covered with a covering member suppressing light reflection.

According to the apparatus for acquiring data for designing a last according to an eighth aspect of the present invention, the heel rest of the present embodiment is attached to the placement section in an attachable and detachable manner, and height of the heel rest is changeable.

### Effects of the Invention

According to the invention of the first aspect, it is possible to accurately measure, without moving a foot during measurement, the data of the sole of the foot that is curved as if footwear such as pumps etc were worn on the foot.

According to the invention of the first aspect, all the pieces of measurement data are acquired at a time using plural cameras, thereby preventing the occurrence of a measurement error caused by a foot movement.

According to the invention of the second aspect, it is possible to more reliably measure the three-dimensional shape of a sole by imaging the sole of a foot placed on the placement section and the heel rest having a certain height, using the first imaging section.

According to the invention of the third aspect, it is possible to irradiate a foot with light without unevenness and prevent the occurrence of a measurement error caused by the brightness /darkness of the light.

According to the invention of the fourth aspect, it is possible to irradiate a sole with light, enabling more accurate measurement of the data of the sole.

According to the invention of the fifth aspect, the placement section further includes a function of irradiating light to a sole, thereby reducing the number of parts of the apparatus for acquiring data for designing a last.

According to the invention of the sixth aspect, the sole illumination table on which a foot is to be placed transmits light irradiated from the sole illumination, thereby irradiating the light to the sole of the foot without unevenness and prevent the occurrence of measurement error caused by the brightness/darkness of the light.

According to the invention of the seventh aspect, the reflection of light irradiated to a sole is suppressed, making it possible to further prevent the measurement error due to the brightness/darkness of the light.

According to the invention of the eighth aspect, with respect to various footwear products having different shapes of heel parts, it is possible to readily measure the data of a sole that is curved in such a state as if footwear is worn thereon.

### BRIEF DESCRIPTION OF THE DRAWINGS

FIG. 1 is a schematic perspective view of an apparatus for acquiring data for designing a last of the present invention.
FIG. 2 is a perspective view of a placement section of the present invention.
FIG. 3 is a perspective view of a vicinity of a second imaging section of the present invention.
FIG. 4 is a perspective view of a vicinity of a third imaging section of the present invention.
FIG. 5 is a block diagram illustrating a configuration of the apparatus for acquiring data for designing a last of the present application.
FIG. 6 is an explanatory diagram of a display section of a control built-in PC of the present invention.
FIG. 7 is an explanatory diagram indicating a method of producing three-dimensional data of the present invention.
FIG. 8 is an explanatory diagram showing specific locations of the cameras which have taken two images in FIG. 7.

### MODE FOR CARRYING OUT THE INVENTION

Described hereinafter with reference to the attached drawings are preferred embodiments of the apparatus for acquiring data for designing a last in the present invention. Throughout all the drawings, common sections are denoted by the same reference numerals.

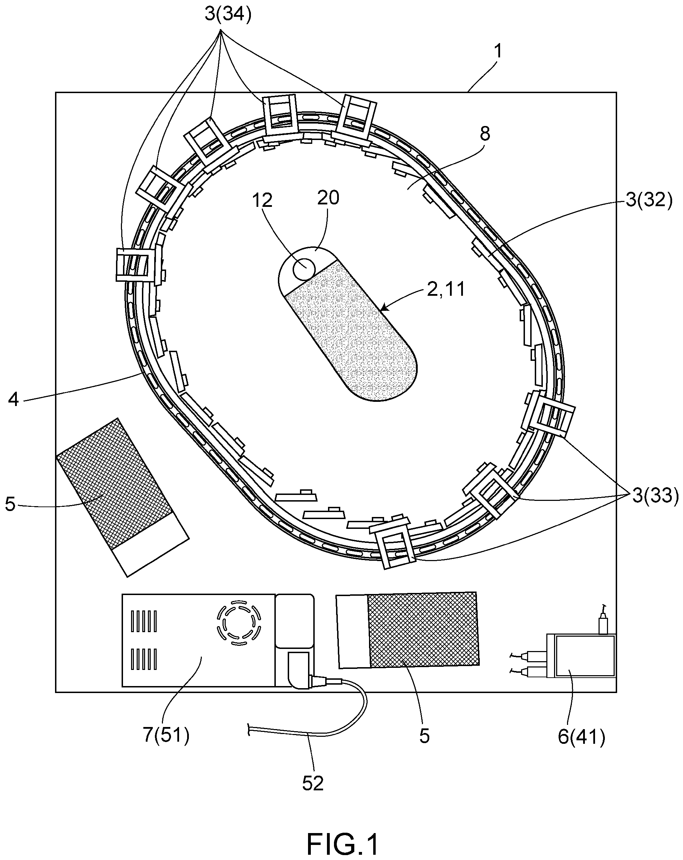

FIG. 1 illustrates an embodiment of the apparatus for acquiring data for designing a last of the present invention.

First, with reference to FIG. 1 is described the whole structure of the apparatus for acquiring data for designing a last. Reference numeral 1 denotes a main body of the apparatus for acquiring data for designing a last. This main body 1 includes a placement section 2 on which a foot F is placed, an imaging means 3 that takes images of the foot F placed on the placement section 2, an entire illumination 4 arranged around the placement section 2, a spoken direction speaker 5, a control built-in PC 6 and a power source 7.

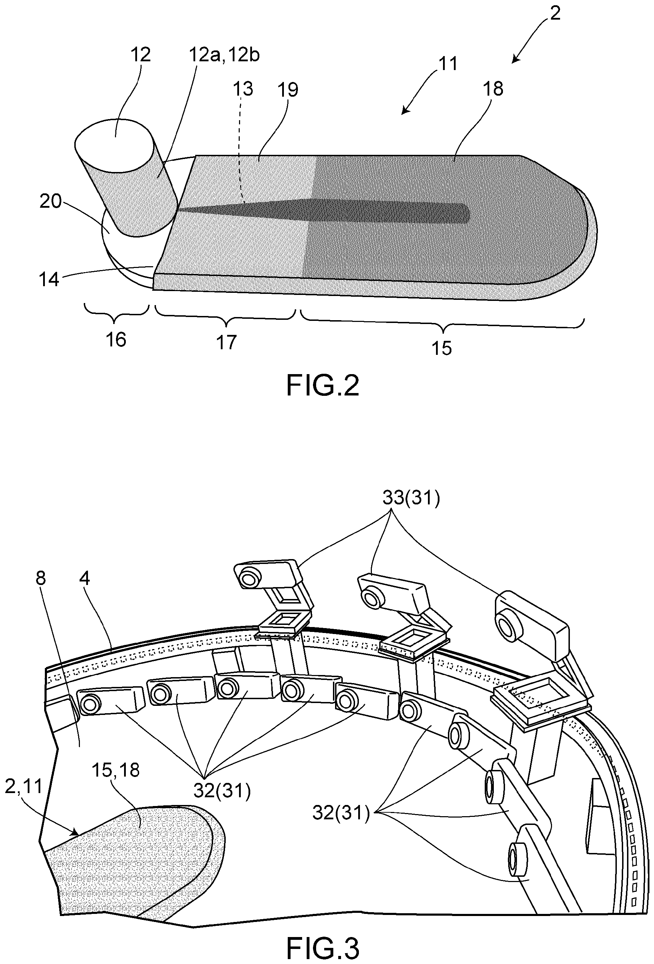

FIG. 2 is a perspective view of the placement section 2 illustrated in FIG. 1. The placement section 2 is composed of a sole illumination table 11 and a heel rest 12. The sole illumination table 11 includes an illumination table body 14 having a light source 13. The sole illumination table 11 irradiates the sole of the foot F placed on the placement section 2 with light, and brings the foot F up to a position higher than a floor part 8, thereby allowing a first imaging section 32 of the imaging means 3 to take an image of the foot F at such an angle that the first imaging section is turned upward. The light source 13 is one having a linear shape, such as an LED, which is arranged along a longitudinal direction of the illumination table main body 14, but is not limited thereto. Although not illustrated here, an illumination table lighting confirmation sensor 21 for confirming whether the light source 13 is turned on, is attached to the sole illumination table 11.

The illumination table main body 14 is made of a plate member having an oval or rectangular shape, which is larger than the area of a sole. Additionally, the illumination table main body 14 is made of a translucent light guiding member such as a translucent acrylic board. Then, one end of the illumination table main body 14 is defined as a toe corresponding section 15 on which a toe is placed, while the other end of the illumination table main body is defined as a heel corresponding section 16 on which a heel is placed, and a middle part between the toe corresponding section 15 and the heel corresponding section 16 is defined as an arch corresponding section 17.

In the illumination table main body 14, the toe corresponding section 15 and the arch corresponding section 17 are respectively provided with a first skin section 18 and a second skin section 19, each of which covers the upper surface of the illumination table main body 14 with a covering member such as cloth. The first skin section 18 is formed so as to be thicker than the second skin section 19. The heel rest 12 is attached to the center of the heel corresponding section 16 in an attachable and detachable manner. The heel corresponding section 16 other than the heel rest 12 serves as a light transmitting section 20 that transmits light from the light source 13. The first skin section 18 and the second skin section 19 are configured such that the first skin section 18 has substantially no translucency, and the second skin section 19 has translucency suppressed as compared with that of the light transmitting section 20.

The heel rest 12 is attached to the sole illumination table 11 in an attachable and detachable manner in order to reproduce the state of the foot F wearing a shoe when the foot F is placed on the placement section 2, and a side surface 12a of the cylindrical body is covered with a covering member 12b such as cloth. It is to be noted that the heel rest 12 is not limited to a cylindrical body as long as it has a shape projecting upward from the upper surface of the illumination table main body 14 and a heel placed thereon is positioned a certain distance away from the upper surface of the illumination table main body 14, and the heel rest 12 may be in the shape of an elliptic cylinder, a prism, a cone, a pyramid, a hemisphere, a truncated cone, a truncated pyramid, other three-dimensional bodies, etc., or a combination of these three-dimensional bodies. Further, the heel rest 12 with a height adjusted to that of the heel part (heel section) of a shoe to be produced, is used and attached to the heel corresponding section 16. Alternatively, the height of the heel rest 12 itself may be configured to be changeable. Such configuration makes it possible to easily measure the data of a sole that is curved in the same manner as when one is actually wearing footwear, with respect to various footwear products having different shapes of heel parts. Further, the first skin section 18, the second skin section 19 and the covering member 12b of the heel rest 12 are each made of a material that suppresses reflection even though it has some light transmitting properties.

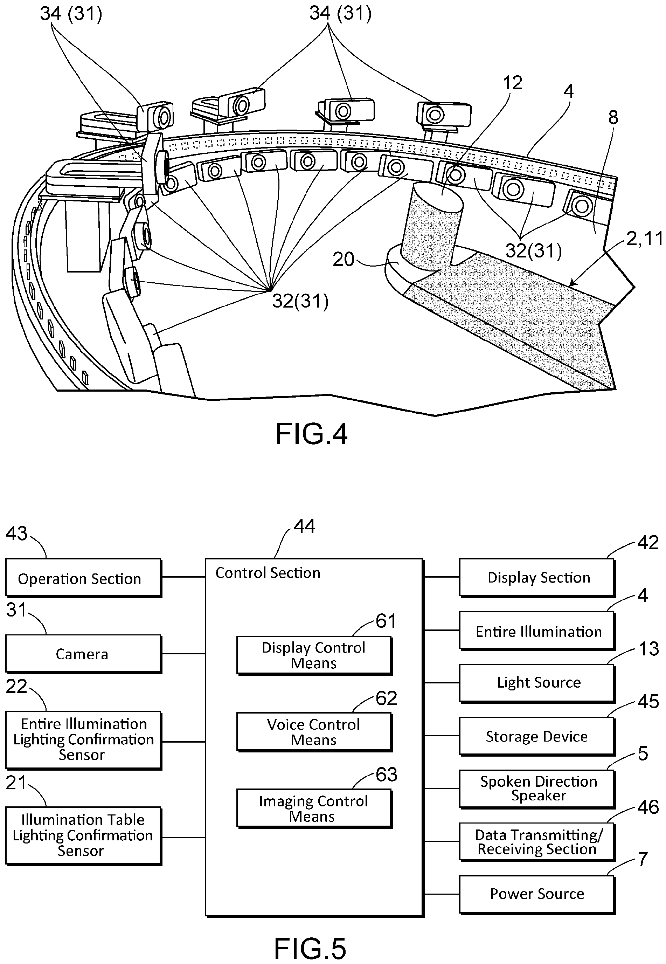

FIGs. 3 and 4 illustrate the arrangement of the imaging means 3 illustrated in FIG. 1. The imaging means 3 includes: a first imaging section 32, in which plural cameras 31 are arranged around the entire circumference of the placement section 2 such that the placement section 2 can be imaged from all directions; a second imaging section 33, as illustrated in FIG. 3, in which the plural cameras 31 are arranged at a higher position than the first imaging section 32 such that the placement section 2 can be imaged from the side of the toe corresponding section 15; and a third imaging section 34, as illustrated in FIG. 4, in which the plural cameras 31 are arranged at a higher position than the first imaging section 32 such that the placement section 2 can be imaged from the side of the heel corresponding section 16. Each camera 31 of the first imaging section 32, the second imaging section 33 and the third imaging section 34 is connected to the control built-in PC 6 through a connection equipment 35 such as a USB hub, so that they can be synchronously operated. Further, in the first imaging section 32, the second imaging section 33 and the third imaging section 34, each camera 31 is arranged such that images taken by the cameras 31 arranged vertically or horizontally adjacent to each other may overlap with each other at a preset ratio. As described above, the first imaging section 32 is configured to image the foot F at such a low angle that one looks up the foot F from the first imaging section 32, the second imaging section 33 is configured to mainly image the instep side of the foot F, and the third imaging section 34 is configured to mainly image the ankle and heel sides of the foot F.

The entire illumination 4 is an illumination appliance such as LED(s) that is configured annularly and continuously so as to surround the entire circumference of the placement section 2. The entire illumination 4 is arranged above the first imaging section 32, while the second imaging section 33 and the third imaging section 34 are arranged above the entire illumination 4. Although not illustrated here, an entire illumination lighting confirmation sensor 22 for confirming that the entire illumination 4 is turned on, is attached to the main body 1.

It is to be noted that a floor section 8 on which the placement section 2, the imaging means 3 and the entire illumination 4 are disposed, is preferably made of a material having the same effect as a reflector plate reflecting the light from the entire illumination 4, such as, for example, a material having a white and smooth surface.

The spoken direction speaker 5 transmits the contents of direction to an operator (a user) with sounds at the time of power-on or imaging.

The control built-in PC 6 is provided with a touch screen 41, and is connected to the Internet through a communication means that is a wired network or a wireless network such as a wireless LAN, a wired LAN, a data line for a mobile phone. The touch screen 41 is used, as sort of an input section of an input terminal, for both a display section 42 and an operation section 43 of the control built-in PC 6. When a user's finger touches and operates the transparent sheet-like operation section 43 disposed on a surface of the display section 42, an operation signal corresponding to a position thus touched is output from the touch screen 41. A display control means that allows the touch screen 41 to perform a desirable display in response to the operation signal from the touch screen 41, is embedded in a control section 44 of the control built-in PC 6.

The power source 7, which is to supply a DC power to each section of the main body 1, is composed of a power source main body 51 and a power cord with a plug 52. The power source main body 51 is connected to the entire illumination 4, the spoken direction speaker 5, the control built-in PC 6, the light source 13 and the cameras 31, respectively.

FIG. 5 is a block diagram illustrating a configuration of the main body 1. The control built-in PC 6 is provided with the control section 44 constituted by, for example, a CPU of a microcomputer, and this control section 44 is connected to the entire illumination 4, the voice announcement means 5, the light source 13, the illumination table lighting confirmation sensor 21, the entire illumination lighting confirmation sensor 22, the cameras 31, the operation section 43, the display section 44, a storage device 45, and the power source main body 51, respectively. Alternatively, the main body 1 may include a data transmitting/receiving section 46, and the data transmitting/receiving section 46 may be connected to the control section 44.

The storage device 45 stores photographic data from all the cameras 31. Further, the storage device 45 stores and retains a program with which a computer allows the control section 44 to function as a display control means 61, a voice control means 62, and an imaging control means 63, respectively.

The display control means 61 controls a display operation of the display section 42, and allows the display section 42 to display a post-power-on screen and a screen corresponding to an operation signal by a key touch to the operation section 43.

The voice control means 62 controls an announcing operation of the spoken direction speaker 5. Further, the voice control means 62 controls the spoken direction speaker 5 to output a voice-guided explanation and a countdown at the time of starting imaging and to play music during imaging.

The imaging control means 63 controls an imaging operation of the camera 31, and controls to memorize and store the photographic data taken by the camera 31 in the storage device 45. Further, the imaging control means 63 may be configured to control the on-off operation of the entire illumination 4 and the light source 13.

The wireless data transmitting/receiving section 46 enables a two-way wired or wireless communication with a three-dimensional data production means 64 through the Internet by the aforementioned communication means, and transmits the photographic data from the cameras 31 to the three-dimensional data production means 64.

It is to be noted that although the main body 1 is not provided with the three-dimensional data production means in the present embodiment, the main body 1 may be provided with such three-dimensional data production means.

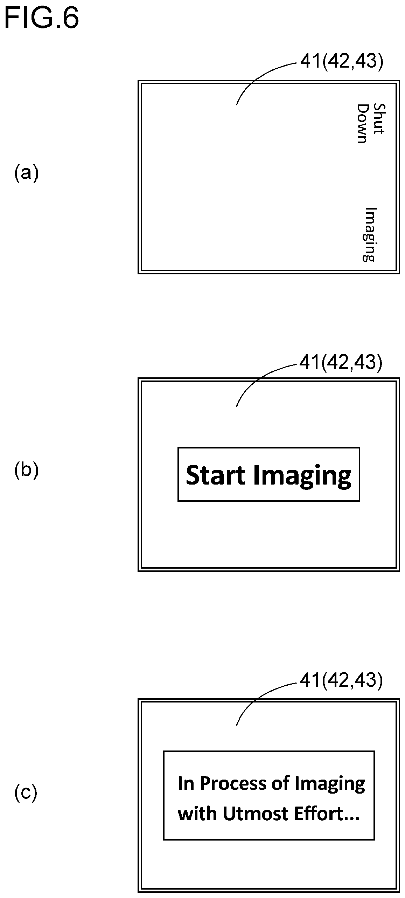

Hereinafter will be described in detail how the configuration of the apparatus for acquiring data for designing a last is operated, with reference to FIG. 6.

To start with, described hereinafter is an operational procedure at the time of power activation. When the power cord with plug 52 of the power source 7 is connected to an AC outlet of a commercial power source, the power source main body 51 supplies a DC power to the entire illumination 4, the spoken direction speaker 5, the control built-in PC 6, the light source 13, and the cameras 31 of the first to third imaging sections 32 to 34. Then, the power sources of the cameras 31 are turned on, so that the entire illumination 4 and the light source 13 are lit, and the control built-in PC 6 is activated. Then, when the control section 44 recognizes all the cameras 31, and confirms that the entire illumination 4 and the light source 13 are lit through the illumination table lighting confirmation sensor 21 and the entire illumination lighting confirmation sensor 22, then a preparation for imaging is completed, and the display control means 61 displays a screen as illustrated in FIG. 6(a) on the display section 42. At this time, the voice control means 62 may be configured to reproduce a sound from the spoken direction speakers 5 and announce that the imaging preparation has been completed.

At this time, when the control section 44 fails to recognize some or all of the cameras 31, or when the control section 44 is incapable of confirming that the entire illumination 4 and the light source 13 are lit through the illumination table lighting confirmation sensor 21 and the entire illumination lighting confirmation sensor 22, then the control section 44 may be configured to automatically reboot the control built-in PC 6. Further, when it is impossible to recognize or confirm the above even if the control built-in PC 6 is rebooted for a predetermined number of times, the display control means 61 may be configured to display on the display section 42 an error message that the camera(s) is/are incapable of being recognized or that lighting of the illumination is incapable of being confirmed.

Next is described an operational procedure at the time of imaging. After completion of the imaging preparation, when one foot F as a photographic subject is placed on the placement section 2, and an "imaging" button displayed on the display section 42 is touched, the display control means 61 allows the display section 42 to display a screen as illustrated in FIG. 6(b). At this time, the voice control means 62 may be configured such that an explanation for the imaging is allowed to come out from the spoken direction speakers 5, and then a countdown starts and the imaging is started after completion of the counting.

When the imaging is started, the imaging control means 63 acquires all the photographic data at a time by using the cameras 31 of the first to third imaging sections 32 to 34 to store them in the storage device 45. At this time, a screen as illustrated in FIG. 6(c) is displayed on the display section 42 to inform the operator and a person to be imaged that the main body 1 is in process of imaging. At this time, in order to make the operator and the person to be imaged feel as if it does not take long for imaging, the voice control means 62 may be configured to play music through the spoken direction speakers 5.

After finishing the imaging of the foot F, if the data transmitting/receiving section 46 is provided in the control built-in PC 6, the control section 44 connects the data transmitting/receiving section 46 to the Internet through the aforementioned communication means, and uploads the photographic data of the foot F from the storage device 45 to send the data to the three-dimensional data production means. Then, the aforementioned imaging ready state will be reached.

Subsequently, the other foot F' is also imaged in the same manner as described above and the photographic data of the foot F' is sent to the three-dimensional data production means.

If the data transmitting/receiving section 46 is not provided in the control built-in PC 6, since the photographic data are stored in the storage device 45, the main body 1 is collected after finishing the imaging of the feet F and F' of the person to be imaged. Then, using an external storage device that is attachable to and detachable from the control built-in PC 6, the photographic data are retrieved from the storage device 45 of the control built-in PC 6, and stored in the three-dimensional data production means.

Described hereinafter is an operational procedure at the time of power off. After finishing the imaging of the feet F and F', the main body 1 comes into an imaging ready state. When a "shut down" button illustrated in FIG. 6(a) is touched, the control section 44 controls the power source main body 51 to stop supplying an electric power to the entire illumination 4, the spoken direction speakers 5, the control built-in PC 6, the light source 13 and the cameras 31 of the first to third imaging sections 32 to 34. At this time, it may be controlled such that supply of an electric power except to the control built-in PC 6 may be promptly stopped, while supply of an electric power to the control built-in PC 6 may be stopped after the control built-in PC 6 itself is shut down.

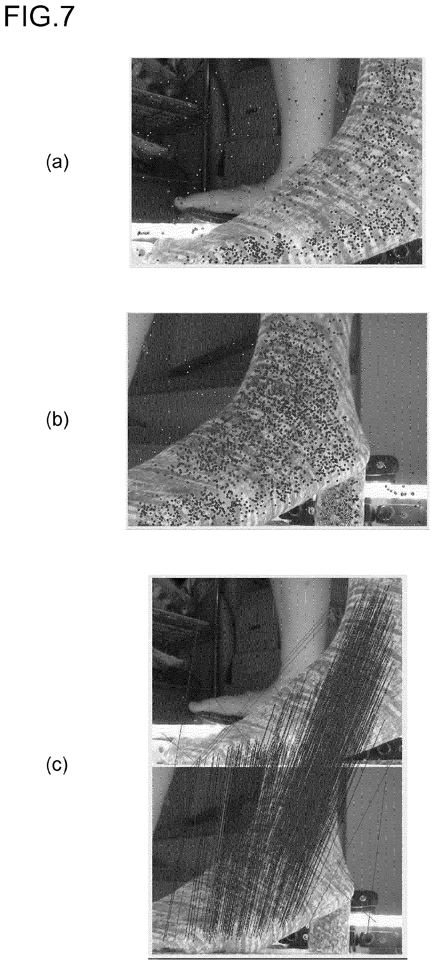

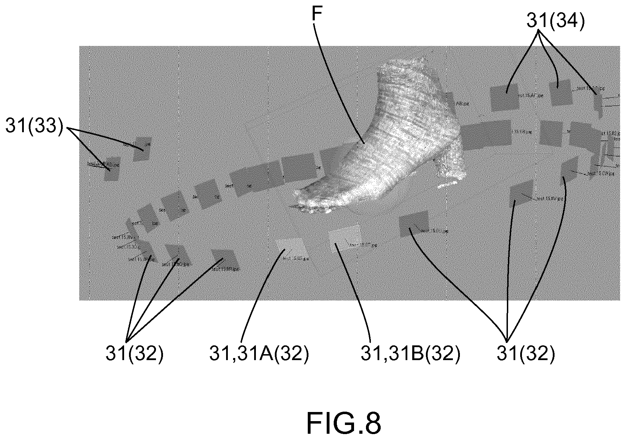

Next, with reference to FIGs. 7 and 8, described hereinafter is a method of producing three-dimensional data in a case where the three-dimensional data production means is provided.

(1) After the image data from the control built-in PC 6 are stored, as illustrated in FIGs. 7(a) and 7(b), a feature point is calculated with respect to all the images taken. A feature point is one that is extracted based on changes in rate of tone change with respect to an arbitrary point and a surrounding point(s) thereof by scanning all the regions in the images. In FIGs. 7(a) and 7(b), such feature points are indicated by dots. Since such feature points are already disclosed in Japanese Patent Application Laid-Open No. 2001-155151 and etc., they will not be described here in detail.
(2) As illustrated in FIG. 7(c), one image is taken out, and feature points in the image and another image that appear to be identical to each other, are connected to each other with lines, followed by confirming all the other points in the same way. In this way, combinations of images having the highest concordance rates of the relative positions of the feature points are identified.
(3) After the identification of the images, the positions of the cameras 31A and 31B which took these two images are calculated based on the pixel positions of the feature points in these two images, as illustrated in FIG. 8.
(4) (2) and (3) are applied to all the images of (1), and thus the positions of all the cameras 31 are determined.
(5) Based on the camera positions determined in (4), the feature points are plotted in a three-dimensional space, thereafter an interpolation calculation is performed to fill spaces between the feature points. After the spaces are filled, a surface is produced and thus three-dimensional data of the feet F and F' to be measured are produced.

As described above, the apparatus for acquiring data for designing a last of the present embodiment is configured to include the placement section 2 for placing the feet F and F' thereon, the imaging means 3 arranged around the placement section 2, wherein the heel rest 12 for placing the heel of the foot F or F' thereon is provided on the placement section 2.

According to the foregoing configuration, it becomes possible to accurately measure the data of a sole that is curved in such a state as if a person to be measured is wearing footwear having a heel, such as a pump, without moving the foot during the measurement.

Further, as the imaging means 3 of the present embodiment, there are provided the first imaging section 32 for imaging the entire circumferences of the feet F and F', the second imaging section 33 for imaging a vicinity of the instep, and the third imaging section 34 for imaging a vicinity of the ankle.

According to such configuration, all the measurement data are acquired at a time using the plural cameras 31, making it possible to prevent measurement errors due to a movement of the foot F or F'.

Also, the first imaging section 32 of the present embodiment is configured to image the feet F and F' placed on the placement section 2 at such an angle that the first imaging section 32 is turned upward.

Such configuration makes it possible to more reliably image, at the first imaging section, the soles of the feet F and F' placed on the placement section 2 and the heel rest 12 having a certain height to measure the three-dimensional shape of the sole.

Further, the apparatus for acquiring data for designing a last of the present embodiment is provided with the entire illumination 4 illuminating the circumference of the placement section 2.

Such configuration makes it possible to uniformly irradiate the foot F or F' with light, and prevent a measurement error due to the brightness /darkness of the light.

Further, the apparatus for acquiring data for designing a last of the present embodiment is provided with the light source 13 as the sole illumination for lighting a sole.

Such configuration makes it possible to irradiate the sole of the foot F or F' with light to thereby more accurately measure the data of the sole.

Further, the placement section 2 of the present embodiment includes the heel rest 12 and the sole illumination table 11 having the light source 13.

Such configuration makes it possible to reduce the number of parts of the apparatus for acquiring data for designing a last since the placement section 2 also has the function of irradiating the sole of the feet F and F' with light.

The sole illumination table 11 of the present embodiment is formed to be larger than a sole area, and has the illumination table main body 14 transmitting light from the light source 13.

Such configuration makes it possible to uniformly irradiate the sole of the foot F or F' with light, and prevent a measurement error due to the brightness/darkness of the light since the illumination table main body 14 on which the foot F or F' is placed transmits light from the light source 13.

Further, the illumination table main body 14 of the present embodiment is configured to be covered with the first skin section 18 and the second skin section 19, which are covering members for suppressing light reflection.

With such configuration, suppressed is the reflection of the light irradiated to the soles of the feet F and F', making it possible to further prevent the measurement error due to the brightness/darkness of the light.

Further, the heel rest 12 of the present embodiment is attached to the placement section 2 in an attachable and detachable manner, and is configured such that the height of the heel rest 12 is changeable.

Such configuration makes it possible to easily measure data of a sole of the foot F or F' that is curved in such a state as if a footwear is being worn thereon with respect to various types of footwear having different shapes of heel parts.

It is to be noted that the present invention is not limited to the above-described embodiment. Without departing from the spirit and scope of the present invention, one of ordinary skill in the art can make various changes and modifications to the invention. For example, in the present embodiment, when the power cord with plug 52 is connected to an AC outlet of a commercial power source, an electric power is supplied to each part of the main body 1. However, a power button may be attached to the power source 7 so that supply of an electric power may be controlled by turning on/off the power source 7. Further, a sensor for detecting the placement of a heel on the heel rest 12 may be provided so that the entire illumination 4, the control built-in PC 6, the light source 13, the camera 31, etc. may be activated when this sensor detects that the heel is placed thereon. Moreover, the configuration, structure and the like of each section are not limited to those of the present embodiment, and may be appropriately changed.

### Description of reference numerals

2. Placement section
3. Imaging means
4. Entire illumination
11. Sole illumination table
12. Heel rest
13. Light source (sole illumination)
14. Illumination table main body
18. First skin section (covering member)
19. Second skin section (covering member)
32. First imaging section
33. Second imaging section
34. Third imaging section

## Claims

1. An apparatus for acquiring data for designing a last comprising:
a main body having a flat plate shape;
a placement section arranged on an upper surface of the main body, the placement section placing a foot thereon;
a first imaging section arranged around the placement section, the first imaging section imaging a whole circumference of the foot;
a second imaging section disposed on the main body, the second imaging section imaging a vicinity of an instep of the foot;
a third imaging section disposed on the main body, the third imaging section imaging a vicinity of an ankle of the foot; and
a heel rest arranged on the placement section, the heel rest placing a heel thereon.

2. The apparatus for acquiring data for designing a last according to claim 1, wherein the first imaging section images the foot placed on the placement section at such an angle that the first imaging section is turned upward.

3. The apparatus for acquiring data for designing a last according to claim 1 or 2, further comprising an entire illumination for illuminating the circumference of the placement section.

4. The apparatus for acquiring data for designing a last according to any one of claims 1 to 3, further comprising a sole illumination for illuminating a sole.

5. The apparatus for acquiring data for designing a last according to claim 4, wherein the placement section is composed of the heel rest and a sole illumination table having the sole illumination.

6. The apparatus for acquiring data for designing a last according to claim 5, wherein the sole illumination table is formed to be larger than an area of the sole, and is provided with an illumination table main body transmitting light from the sole illumination.

7. The apparatus for acquiring data for designing a last according to claim 6, wherein the illumination table main body is covered with a covering member suppressing light reflection.

8. The apparatus for acquiring data for designing a last according to any one of claims 1 to 7, wherein the heel rest is attached to the placement section in an attachable and detachable manner so that height of the heel rest height is made changeable.
